# EUROPEAN PATENT APPLICATION

(11) **EP 1 949 962 A1**
(43) Date of publication of application: **30.07.2008**
(21) Application number: 07001572.2
(22) Date of filing: 25.01.2007
(51) Int. Cl.: B01L 3/14, B01L 3/00, G01N 21/07

(54) **Device and method for separating a liquid component of a blood sample, and analyzer apparatus comprising such a device**

(71) Applicant: F.HOFFMANN-LA ROCHE AG, 4070 Basel (CH); Roche Diagnostics GmbH, 68305 Mannheim (DE)
(72) Inventor: Wahl, Hans Peter, 79650 Schopfheim (DE); Burkhardt, Claudius, 6004 Luzern (CH)
(74) Representative: Ventocilla, Abraham

(57) **Abstract**

A device for separating and collecting a liquid component of a blood sample. The device comprises
(a) a container body (11, 41) having a symmetry axis (13, 43) and consisting of
(a.1) an upper portion (14, 44) having a cylindrical side wall (15, 45), and an upper opening (16, 46), and
(a.2) a lower portion (17, 47) having a bottom region (18, 48) and a bottom wall (19, 49), and

(c) a porous material (24, 84) arranged within said container body (11), the outer surface of said porous material (24, 84) being in contact with and covering at least partially the inner surface of said cylindrical side wall (15, 85), said porous material (24, 84) being suitable for retaining non-liquid components (32) of said blood sample (31).

## Description

### Field of the invention

The invention concerns a device for separating a liquid component of a blood sample.

The invention also concerns a method for separating a liquid component of a blood sample.

The invention further concerns an analyzer apparatus comprising a device of the above mentioned kind.

### Background

The separation of liquid components of blood samples, e.g. plasma and serum, is a necessary and important part of the preparation of those samples for their processing in modern clinical diagnostic analyzer apparatuses.

Known methods for performing the above mentioned separation require an important amount of manual work and time. The automation of that manual work would require a high degree of robotic automisation. Moreover the most widely used separation methods are batch processes, i.e. treatment of individual samples is not possible.

There is therefore a need for a device and a method which overcome the above mentioned drawbacks of prior art in this field.

### Summary of the invention

A first aim of the invention is to provide a device for separating a liquid component of a blood sample which allows overcoming the above mentioned drawbacks.

According to a first aspect of the invention the above first aim is achieved by means of a device defined by claim 1.

Claims 2, 3, 6 to 12 and 18 to 25 define preferred embodiments of this device.

A second aim of the invention is to provide a method for separating a liquid component of a blood sample which allows overcoming the above mentioned drawbacks.

According to a second aspect of the invention the above second aim is achieved by methods defined by claims 4, 13, 14, 26 and 27. Claims 15 and 28 define preferred embodiments of those methods.

A third aim of the invention is to provide a clinical diagnostic analyzer apparatus which comprises means for separating a liquid component of a blood sample.

According to a third aspect of the invention the above third aim is achieved by means of analyzer apparatuses defined by claims 5, 16, 17, 29 and 30.

The main advantages obtained with a device and a method according to the invention are as follows:
The time required for plasma or serum separation is substantially reduced, because the length of the motion path followed by cell components of blood sample during the axial centrifugation of the sample container by rotation thereof about its symmetry axis is much shorter than in a conventional swing bucket centrifugation of a primary sample tube. When swing bucket centrifugation of a sample tube containing a blood sample is performed the rotation axis is perpendicular to the length axis of the sample tube and the motion path followed by cell components of blood sample during the centrifugation is much longer because the cell components move along the length axis of the sample tube towards the bottom of this tube.

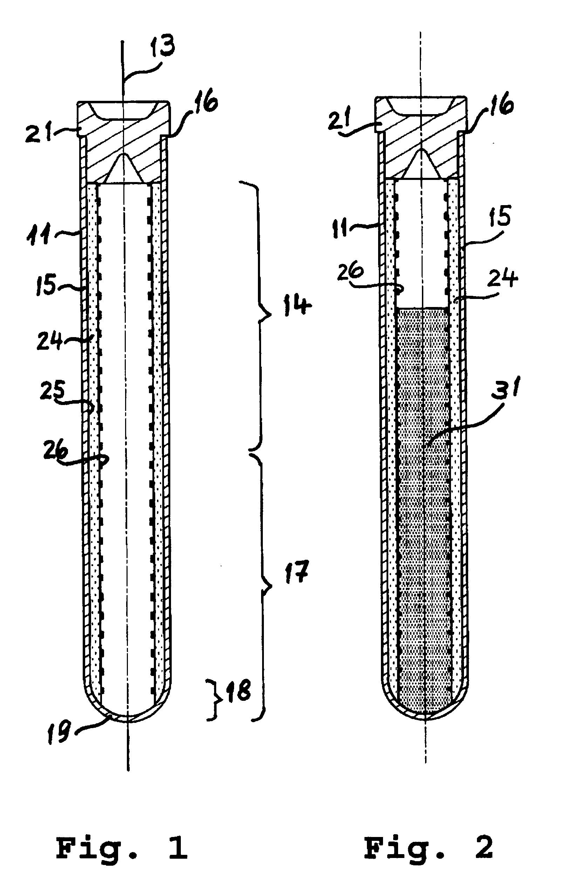

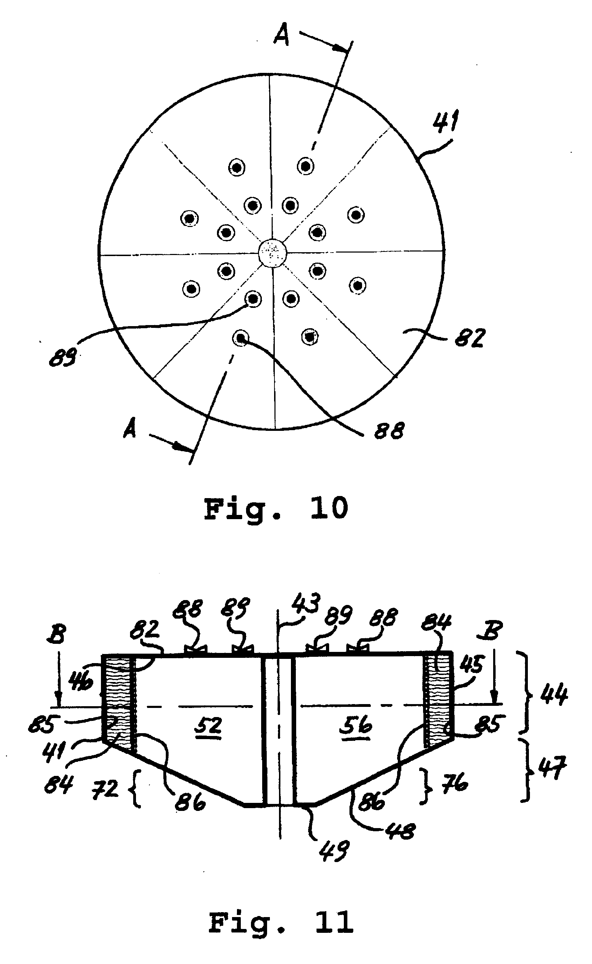

The centrifugation time necessary for achieving plasma or serum separation with a device according to the invention is shorter than the centrifugation time required in the case of a conventional swing bucket centrifugation of a primary sample tube, because the cell components of the blood sample have to migrate along a shorter distance. In the embodiments shown by Figures 1 to 9, wherein the sample container is a sample tube, the migration distance of the cell components of the blood sample is less than half the length of the radius of the cross-section of the sample container. In the embodiments shown by Figures 10 to 17, the migration distance of the cell components of the blood sample is only about half the distance of the thickness of the layer of blood which is spun to the outer wall of the sample container.

If a primary sample tube is used as a sample container for axial centrifugation according to the invention, the length of the motion path followed by cell components of blood sample during axial centrifugation is much less than the length of the radius of a the primary sample tube, e.g. between 1 and 4 mm.

The centrifuge required for axial centrifugation of a single sample container is small and is thus suitable for being integrated as an automatically operated centrifuge into an analyzer apparatus even if the space available is relatively small. Such apparatus preferably comprises one such centrifuge for all of the sample containers in the analyzer apparatus.

Plasma or serum separated by axial centrifugation of a sample container according to the invention is taken out of the sample container by means of the pipetting needle of the automatic pipetting unit of an analyzer apparatus. For this purpose the pipetting needle pierces the plug which closes a primary sample tube if such a tube is used as sample container for axial centrifugation. This process is thus much simpler and faster than the conventional method which requires an additional plasma separating device which has to be manually introduced into the sample tube or uses a built in gel in the primary sample tube. Such a gel is relatively expensive and with this latter method it takes relatively long time, e.g. about 15 minutes, to separate the liquid component from the non-liquid components of the blood sample.

A preferred embodiment of the sample container includes a cup which is suitable for performing photometric or reflectometric measurements of serum or plasma separated from a blood sample. This embodiment makes thus possible to perform sample integrity checks including measurement of serum indices.

The above mentioned advantages make it possible to separate a liquid component of a whole blood sample, e.g. plasma or serum, automatically and exclusively with components available in the analyzer apparatus. In this way, all required integrity checks of the blood sample can be carried out automatically in the analyzer apparatus before the usual analysis of plasma or serum samples is carried out in the analyzer apparatus. The compartmented device defined by claims 18 to 25 makes possible to treat simultaneously a plurality of blood samples in a single container, i.e. to separate the liquid components of those samples with a single centrifugation step.

### Brief description of the drawings

The subject invention will now be described in terms of its preferred embodiments with reference to the accompanying drawings. These embodiments are set forth to aid the understanding of the invention, but are not to be construed as limiting.
Fig. 1 is a cross-sectional view of a first embodiment of a device according to the invention, the cross-section being taken along a plane passing through the symmetry axis 13 of a sample tube 11.
Fig. 2 is a cross-sectional view of the sample tube 11 like the one shown by Fig. 1 and of a blood sample 31 contained in sample tube 11.
Fig. 3 is a cross-sectional view of the sample tube 11 like the one shown by Fig. 1 at the end of the axial centrifugation of the sample tube 11, the centrifugation being effected by spinning sample tube 11 about its symmetry axis 13. Fig. 3 schematically shows the separation of the blood sample into its non-liquid components 32 and its liquid component 33.
Fig. 4 is a cross-sectional view of the sample tube 11 like the one shown by Fig. 1 after axial centrifugation of the sample tube 11 and with this tube at rest.
Fig. 5 is a cross-sectional, enlarged view of sample tube 11 shown by Fig. 3, the cross-sectional view being taken along a plane perpendicular to the symmetry axis of sample tube 11. Fig. 5 shown more in detail the spatial distribution of the non-liquid components 32 and the liquid component 33 of a blood sample at the end of the axial centrifugation of the sample tube 11,
Fig. 6 is a cross-sectional view of a variant of the embodiment shown by Fig. 1 which comprises a cup 22 which is fluidically connected with the interior of sample tube 11 through an opening 23 in the bottom wall 19 and which is suitable for performing photometric measurements.
Fig. 7 is a cross-sectional view of the sample tube 11 like the one shown by Fig. 6 and of a blood sample 31 contained in sample tube 11.
Fig. 8 is a cross-sectional view of the sample tube 11 like the one shown by Fig. 6 at the end of the axial centrifugation of the sample tube 11. Fig. 8 schematically shows the separation of the blood sample into its non-liquid components 32 and its liquid component 33.
Fig. 9 is a cross-sectional view of the sample tube 11 like the one shown by Fig. 6 after axial centrifugation of the sample tube 11 and with this tube at rest.
Fig. 10 shows a top plan view of a second embodiment of a device according to the invention.
Fig. 11 shows a cross-sectional view of the device shown in Fig. 10 taken along a plane A-A.
Fig. 12 shows a cross-sectional view of the device shown in Fig. 10 taken along a plane B-B in Fig. 11.
Fig. 13 shows a cross-sectional view of the device shown in Fig. 10 taken along a plane A-A and after blood samples have been loaded into compartments 52 and 56 of this device and with this device at rest.
Fig. 14 shows a cross-sectional view of the device shown in Fig. 10 taken along a plane A-A and at the end of the axial centrifugation of container body 41, the centrifugation being effected by spinning the container body 41 about its symmetry axis 43.
Fig. 15 shows a cross-sectional view of the device shown in Fig. 1 taken along a plane A-A after axial centrifugation of this device and with this device at rest.
Fig. 16 shows a cross-sectional view of a variant of the device shown in Fig. 10. Fig. 16 shows cups 102 and 106 which are fluidically connected with compartments 52 and 56 respectively and which are suitable for performing photometric measurements.
Fig. 17 shows a front view of a variant of the device shown in Fig. 16. Fig. 17 shows only one of the compartments of this variant and shows a cup 132 which is fluidically connected with a compartment 52 and which is suitable for performing photometric measurements.

### Reference numerals used in drawings

- 11: primary sample tube
- 13: symmetry axis of sample tube 11 / rotation axis for
- axial: centrifugation of sample tube 11
- 14: upper portion of sample tube 11
- 15: cylindrical side wall of sample tube 11
- 16: upper opening of sample tube 11
- 17: lower portion of sample tube 11
- 18: bottom region of sample tube 11
- 19: bottom wall of sample tube 11
- 21: cap of sample tube 11
- 22: cup connected with the interior of sample tube 11
- 23: opening in the bottom wall 16 of sample tube 11
- 24: layer of porous material
- 24a: outer annular portion of layer 24
- 24b: inner annular portion of layer 24
- 25: inner surface of cylindrical side wall 15
- 26: tubular mesh or stent
- 27: air
- 31: blood sample
- 32: non-liquid components of blood sample 31
- 33: liquid component of blood sample 31
- 41: container body
- 43: symmetry axis of container body 41 / rotation axis for
- axial: centrifugation of container body 41
- 44: cylindrical upper portion of container body 41
- 45: cylindrical side wall of container body
- 46: upper opening of container body 41
- 47: lower portion of container body 41
- 48: conical side wall of container body 41
- 49: bottom wall of container body 41
- 50: arrow
- 51-58: compartment of container body 41
- 61-68: partition wall of container body 41
- 72: bottom region of compartment 52
- 76: bottom region of compartment 56
- 82: cover of upper opening 46 of container body 41
- 84: layer of porous material
- 84a: outer portion of layer 84
- 84 b: inner portion of layer 84
- 85: inner surface of side wall 45
- 86: mesh / stent
- 87: air
- 88: pipetting opening / piercable pipetting opening
- 89: pipetting opening / piercable pipetting opening
- 92: opening in the bottom wall of compartment 52
- 96: opening in the bottom wall of compartment 56
- 102: cup connected with compartment 52
- 106: cup connected with compartment 56
- 112: mirror arranged within cup 102
- 116: mirror arranged within cup 106
- 121: incident light beam
- 122: reflected light beam
- 123: incident light beam
- 124: reflected light beam
- 130: arrow
- 132: cup connected with compartment 52
- 141: mirror
- 142: mirror
- 143: incident light beam
- 144: reflected light beam
- 145: reflected light beam
- 312: blood sample in compartment 52
- 316: blood sample in compartment 56
- 322: non-liquid component of blood sample 312
- 326: non-liquid component of blood sample 316
- 332: liquid component of blood sample 312
- 336: liquid component of blood sample 316

### Detailed description of the invention

Preferred embodiments are described hereinafter with reference to the accompanying drawings.

In general terms a device according to the invention for separating and collecting a liquid component of a blood sample comprises a container body and a porous material arranged within the container body. The container body has a symmetry axis and consists of an upper portion having a cylindrical side wall and an upper opening, and a lower portion having a bottom region and a bottom wall. The outer surface of the porous material is in contact with and covers at least partially the inner surface of the cylindrical side wall of the upper portion of the container body. The porous material is suitable for retaining non-liquid components 32 of the blood sample 31.

In a preferred embodiment the container body of the device is a one-piece container body.

In a preferred embodiment, the above mentioned device further comprises a mesh or stent which is in contact with and extends at least partially over the inner surface of the layer of porous material.

In general terms a method according to the invention for separating and collecting a liquid component of a blood sample comprises
(a) introducing a blood sample into container body of a device of the above described type,
(b) rotating said container body about its symmetry axis at a predetermined speed for separating the liquid component of the blood sample from the non-liquid components thereof, and
(c) stopping said rotating of said container body, thereby allowing the liquid component of the blood sample to flow towards the central and lower part of the container body, whereas the entire non-liquid components of the blood sample and a portion of the liquid component of the blood sample are retained by the layer of porous material.

In general terms a clinical diagnostic analyzer apparatus according to the invention comprises
(a) a device of the above described type, and
(b) means for centrifugating the container body of said device about its symmetry axis (13, 43).

### FIRST EXAMPLE OF A DEVICE ACCORDING TO THE INVENTION

A first example of a device according to the invention is described hereinafter with reference to Fig. 1.

As shown by Fig. 1, in this first example the container body of the device is a sample tube 11 and a layer 24 of a porous material is arranged within sample tube 11.

Sample tube 11 has a symmetry axis 13, a cylindrical side wall 15, an upper opening 16, a bottom region 18, and a bottom wall 19. The upper opening 16 of sample tube 11 is closed by a cap 21 which is piercable by a pipetting needle. The interior of sample tube 11 comprises an upper portion 14 and a lower portion 17.

The outer surface of layer 24 is in contact with and covers at least partially the inner surface 25 of the cylindrical side wall 15. In a preferred embodiment porous material layer 24 covers the entire inner surface 25 of the cylindrical side wall 15.

Layer 24 of porous material is suitable for retaining non-liquid components 32 of the blood sample 31.

Layer 24 of porous material is made of e.g. an open cell foam, a foamed rubber, a fleece, a mat, a honeycomb-like material or the like.

In a preferred embodiment the device comprises in addition a tubular mesh or stent 26 which is in contact with and extends at least partially over the inner surface of layer 24 of porous material. In a preferred embodiment, tubular mesh or stent 26 extends over the entire inner surface of layer 24 of porous material.

Tubular mesh or stent 26 is made preferably of a plastic material, e.g. Nylon, Teflon or the like. Tubular mesh or stent 26 keeps layer 24 in place, ensures a better down flow of the liquid component of the blood sample and improves the stability of the physical separation of the liquid component and the non-liquid components of the blood sample.

### FIRST EXAMPLE OF A METHOD ACCORDING TO THE INVENTION

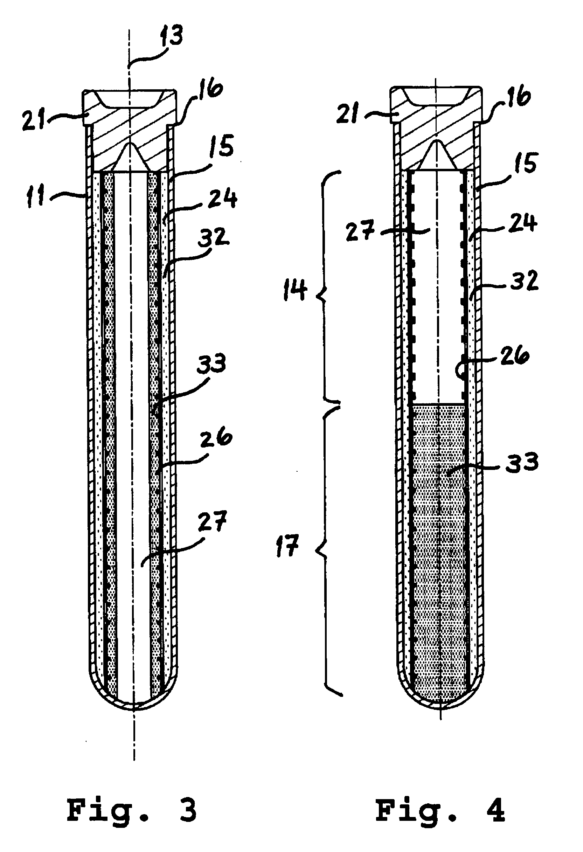

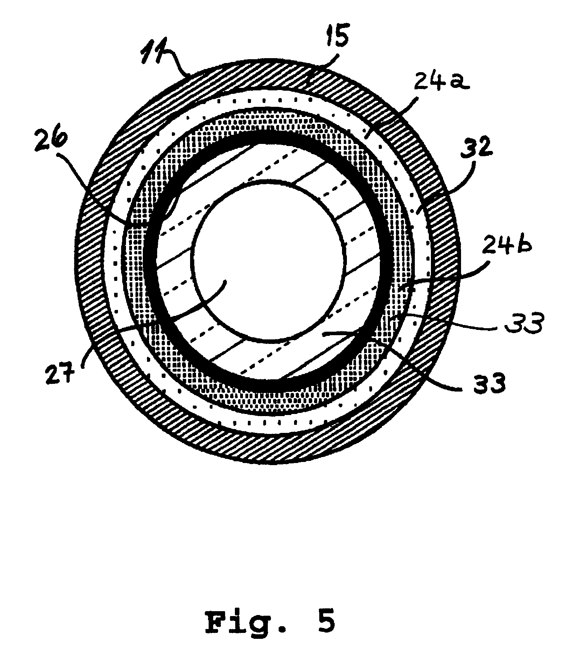

A first example of a method according to the invention for separating a liquid component from a blood sample makes use of the device described above with reference to Fig. 1 and comprises the following steps illustrated by Figures 2 to 5:
(a) introducing a blood sample 31 (shown in Fig. 2) into the sample tube 11,
(b) rotating sample tube 11 about its symmetry axis 13 (as shown by Figures 3 and 5) at a predetermined speed for separating the liquid component of the blood sample from the non-liquid components thereof, and
(c) stopping the rotating of sample tube 11, thereby allowing the liquid component of the blood sample to flow towards the central and lower part of the sample tube 11, whereas the entire non-liquid components of the blood sample 31 and a portion of the liquid component of the blood sample are retained by layer 24 of the porous material.

Step (b) is carried out with a rotation speed adjusted to a value in a range between 1000 and 20000 rpm. The time required for the separation depends from the rotation speed. A decrease of the time required for the separation is obtained by increasing the rotation speed. In the case of centrifugation of the sample tube 11 the following are examples of rotation speeds used and of the values of the separation time achieved:

| | | |
|---|---|---|
| Rotation speed | 1000 rpm | 20000 rpm |
| Separation time | 20 minutes | 15 seconds. |

With a rotation speed of 20000 rpm, separation of platelet-free plasma of a blood sample is achieved in a separation time lying in a range from 30 to 60 seconds.

After step (c) a portion of the liquid component of the blood sample can be collected by pipetting through the stopper 21 while the entire non-liquid components of the blood sample 31 and a portion of the liquid component of the blood sample are retained by layer 24 of the porous material.

The cross-sectional view shown by Fig. 5 shows the spatial distribution of the liquid component 33 and the non-liquid components 32 of blood sample 31 at the end of the axial centrifugation of sample tube 11 according to step (b) but before stopping the rotation of the above described method and as schematically represented by Fig. 3. As shown by Fig. 5, the non-liquid components 32 occupy an outer annular portion 24a of layer 24 of porous material, whereas a first portion of the liquid component 33 occupies an inner annular portion 24b of layer 24 of porous material and a second portion of the liquid component 33 occupies an annular space between tubular mesh 26 and a space 27 occupied by air in the interior of sample tube 11.

In one embodiment of the above described method, sample tube 11 is a blood collection tube that contains a vacuum, this tube contains a coagulation preventing agent, and the blood sample 31 is introduced into the blood collection tube by venipuncture. In this case the liquid component 33 separated by the above described method is blood plasma.

In another embodiment of the above described method, sample tube 11 is a blood collection tube that contains a vacuum, this tube contains no coagulation preventing agent or contains a coagulation promoting agent, and the blood sample 31 is introduced into the blood collection tube by venipuncture. In this case the liquid component 33 separated by the above described method is blood serum.

After step (c) sample tube 11 and the separated liquid component 33 contained therein are usually kept at a suitable temperature until sample tube 11 and its contents are transferred to a clinical diagnostic analyzer apparatus for analysis of the liquid component 33. For this purpose, the liquid component 33 of the blood sample is pipetted from sample tube 11 by means of a pipetting needle which pierces cap 21, enters sample tube 11 and aspirates the liquid component 33, which is transferred e.g. to a reaction cuvette of the clinical diagnostic analyzer apparatus.

### SECOND EXAMPLE OF A DEVICE ACCORDING TO THE INVENTION

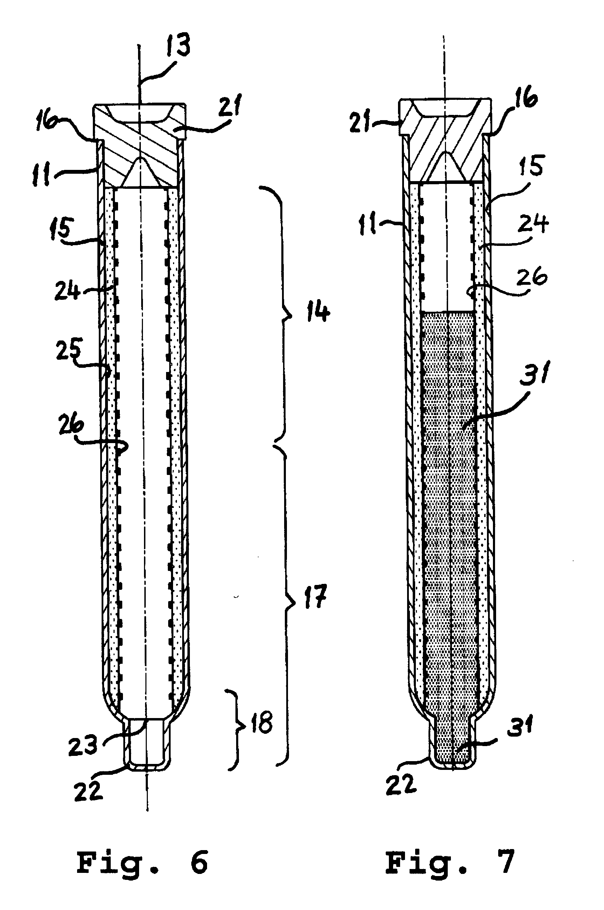

A second example of a device according to the invention is described hereinafter with reference to Fig. 6.

The structure of this second example is a variant of the structure of the example shown by Fig. 1. In this variant, sample tube 11 shown by Fig. 6 comprises in addition a cup 22, which is fluidically connected with the interior of sample tube 11 through an opening 23 in the bottom wall 19 of sample tube 11. Cup 22 is configured and dimensioned for enabling photometric measurement of a liquid contained in cup 22.

### SECOND EXAMPLE OF A METHOD ACCORDING TO THE INVENTION

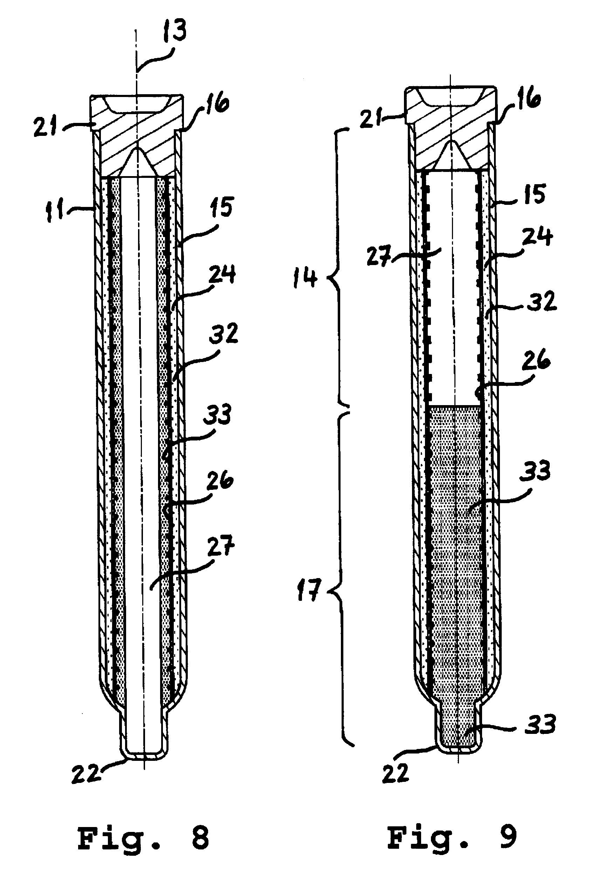

A second example of a method according to the invention for separating a liquid component from a blood sample makes use of the device described above with reference to Fig. 6 and comprises the following steps illustrated by Figures 7 to 9:
(a) introducing a blood sample 31 (shown in Fig. 7) into sample tube 11 (shown in Fig. 6),
(b) rotating sample tube 11 about its symmetry axis 13 at a predetermined speed for separating the liquid component of the blood sample 31 from the non-liquid components thereof (as shown by Fig. 8), and
(c) stopping the rotating of sample tube 11, thereby allowing the liquid component of the blood sample to flow towards the central and lower part of the sample tube 11 and into cup 22, whereas the entire non-liquid components 32 of the blood sample 31 and a portion of the liquid component 33 of the blood sample are retained by layer 24 of the porous material.

Step (b) is carried out with a rotation speed adjusted to a value in a range between 1000 and 20000 rpm. The time required for the separation depends from the rotation speed. A decrease of the time required for the separation is obtained by increasing the rotation speed. In the case of centrifugation of the sample tube 11 the following are examples of rotation speeds used and of the values of the separation time achieved:

| | | |
|---|---|---|
| Rotation speed | 1000 rpm | 20000 rpm |
| Separation time | 20 minutes | 15 seconds. |

With a rotation speed of 20000 rpm, separation of platelet-free plasma of a blood sample is achieved in a separation time lying in a range from 30 to 60 seconds.

After step (c) a portion of the liquid component of the blood sample can be collected by pipetting through the stopper 21 while the entire non-liquid components of the blood sample 31 and a portion of the liquid component of the blood sample are retained by layer 24 of the porous material.

In this second example of a method according to the invention a portion of the liquid component 33 separated by the above described method is contained in cup 22 and enables photometric measurements of the liquid component 33 contained in this cup.

In one embodiment of the above described method, sample tube 11 is a blood collection tube that contains a vacuum, this tube contains a coagulation preventing agent, and the blood sample 31 is introduced into the blood collection tube by venipuncture. In this case the portion of the liquid component 33 separated by the above described method and contained in cup 22 is blood plasma.

In another embodiment of the above described method, sample tube 11 is a blood collection tube that contains a vacuum, this tube contains no coagulation preventing agent or contains a coagulation promoting agent, and the blood sample 31 is introduced into the blood collection tube by venipuncture. In this case the portion of the liquid component 33 separated by the above described method and contained in cup 22 is blood serum.

In this case, sample integrity checks including measurement of serum indices can be performed by photometric measurement of the serum sample contained in cup 22.

The further processing of sample tube 11 after step (c) is e.g. as described above in the first example of a method according to the invention.

### FIRST EXAMPLE OF AN ANALYTICAL APPARATUS ACCORDING TO THE INVENTION

A first example of an analytical apparatus according to the invention is e.g. a clinical diagnostic analyzer apparatus comprising a device as described above with reference to Fig. 1 and an arrangement for centrifugating the sample tube 11 of such a device about its symmetry axis 13.

In a preferred embodiment of this apparatus it comprises a device as described above with reference to Fig. 6 and electro-optical means for performing photometric measurements of the contents of the cup 22 of that device.

### THIRD EXAMPLE OF A DEVICE ACCORDING TO THE INVENTION

A third example of a device according to the invention is described hereinafter with reference to Figures 10 to 12.

Fig. 10 shows a top plan view of a second embodiment of a device according to the invention.

Fig. 11 shows a cross-sectional view of the device shown in Fig. 10 taken along a plane A-A.

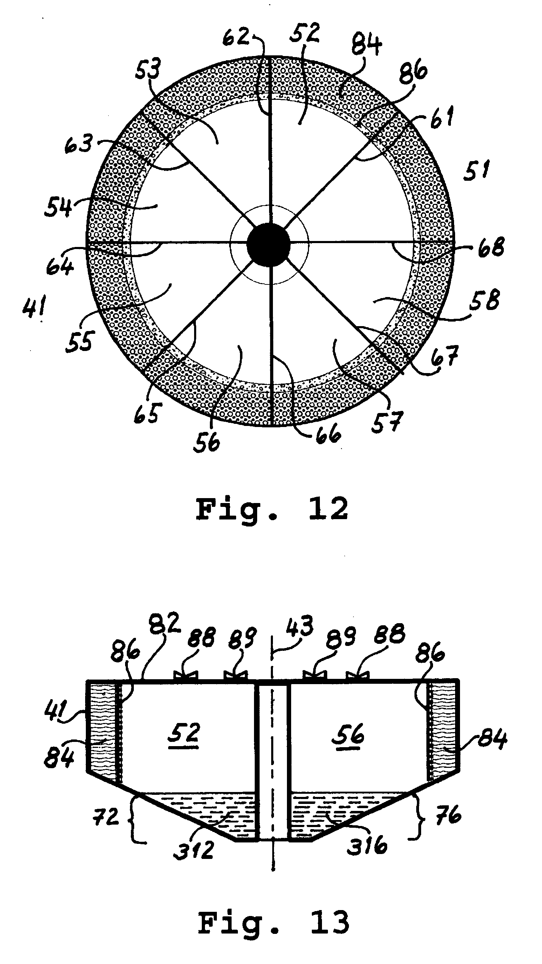

Fig. 12 shows a cross-sectional view of the device shown in Fig. 10 taken along a plane B-B in Fig. 11.

As shown by Figures 10 to 12, the third example of a device according to the invention comprises a container body 41 which has a symmetry axis 43. As shown by Fig. 12, the interior of container body 41 is divided into a plurality of compartments 51 to 58 by radially oriented partition walls 91 to 98. Each of compartments 51 to 58 is adapted for receiving a blood sample. All compartments 51 to 58 have preferably the same shape and the same size, and the number of compartments is preferably an even number. In a preferred embodiment the container is divided into 12 compartments. Each one of compartments 51 to 58 has a bottom region like 72 and 76 shown in Fig. 11. In a preferred embodiment container body 41 is a one-piece container body.

Container body 41 has an upper portion 44 and a lower portion 47. The upper portion 44 of container body 41 has a cylindrical side wall 45 and an upper opening 46. The lower portion 47 of container body 41 has the shape of a truncated cone and has a conical side wall 48 and a bottom wall 49.

The device shown by Figures 10 to 12 further comprises a layer 84 of a porous material which is arranged within each of compartments 51 to 58. The outer surface of layer 84 is in contact with and covers at least partially the inner surface 85 of the portion of the cylindrical side wall 45 which belongs to that compartment. In a preferred embodiment porous material layer 84 covers the entire inner surface of the portion of the cylindrical side wall 45 which belongs to each compartment 51 to 58.

Layer 84 of porous material is suitable for retaining non-liquid components 32 of the blood sample 31.

The device shown by Figures 10 to 12 further comprises a cover 82 of the upper opening 46 of container body 41. Cover 82 is permanently connected to container body 41. Cover 82 has at least one pipetting opening 89 for each of the compartments 51 to 58. The at least one pipetting opening 89 allows passage of pipetting needle therethrough for pipetting a blood sample into or out of one of compartments 51-58. In a preferred embodiment cover 82 has a first pipetting opening 88 and a second pipetting opening 89 for each of the compartments 51 to 58. First pipetting opening 88 allows passage of a pipetting needle therethrough for pipetting a blood sample into one of compartments 51-58. Second pipetting opening 89 allows passage of a pipetting needle therethrough for pipetting out of one of compartments 51-58 a liquid component separated from that blood sample.

Layer 84 of porous material is made of e.g. an open cell foam, a foamed rubber, a fleece, a mat, a honeycomb-like material or the like.

In a preferred embodiment the device shown by Figures 10 to 12 comprises in addition a mesh or stent 86 which is in contact with and extend at least partially over the inner surface of layer 84 of porous material. In a preferred embodiment, mesh or stent 86 extends over the entire inner surface of layer 84 of porous material.

Mesh or stent 86 is made preferably of a plastic material, e.g. nylon or Teflon or the like. Mesh 86 keeps layer 84 in place, ensures a better down flow of the liquid component of the blood sample and improves the stability of the physical separation of the liquid component and the non-liquid components of the blood sample.

### THIRD EXAMPLE OF A METHOD ACCORDING TO THE INVENTION

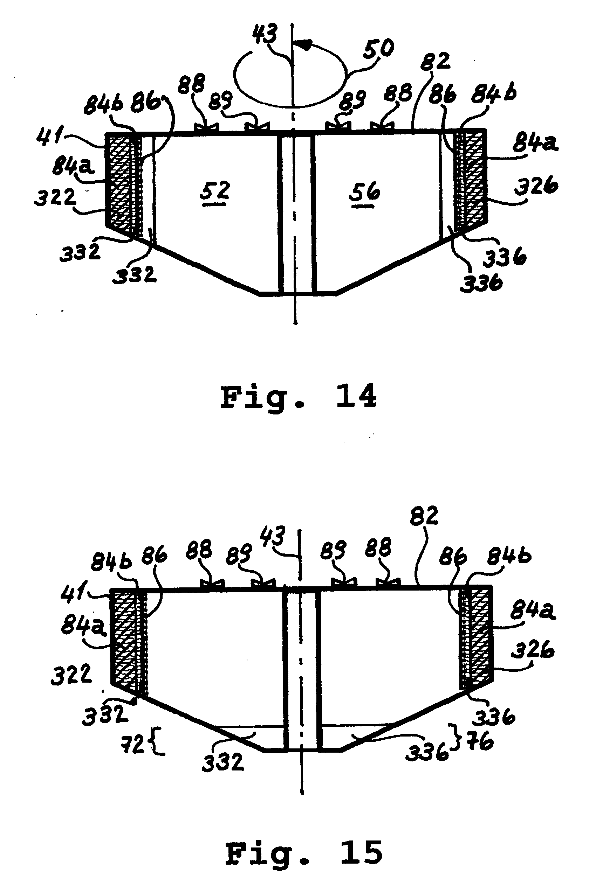

An third example of a method according to the invention for separating a liquid component from a blood sample makes use of the device described above with reference to Figures 10 to 12 and comprises the following steps illustrated by Figures 13 to 15 which show compartments 52 and 56 of container body 41:
(a) introducing blood samples 312, 316 (shown in Fig. 13) into respective compartments 52, 56 of the container body 41 of a device according to the above described third example of a device according to the invention,
(b) rotating the container body 41 about its symmetry axis 43 (as shown by Fig. 14) at a predetermined speed and in a sense shown by arrow 50 for separating the liquid components 332, 336 of the blood samples 312, 316 from the non-liquid components 322, 326 thereof, and
(c) stopping the rotating of the container body 41, thereby allowing the liquid components 332, 336 of the blood samples 312, 316 to flow towards the central and bottom regions 72, 76 of the respective compartments 52 respectively 56 of the container body 41, whereas the entire non-liquid components 322, 326 of the blood samples 312, 316 and portions of the liquid components 332, 336 of the blood samples 312, 316 are retained by layer 84 of the porous material.

Step (b) is carried out with a rotation speed adjusted to a value in a range between 500 and 10000 rpm. The time required for the separation depends from the rotation speed. A decrease of the time required for the separation is obtained by increasing the rotation speed. In the case of centrifugation of the container body 41 the following are examples of rotation speeds used and of the values of the separation time achieved:

| | | |
|---|---|---|
| Rotation speed | 500 rpm | 10000 rpm |
| Separation time | 20 minutes | 15 seconds. |

With a rotation speed of 10000 rpm, separation of platelet-free plasma of a blood sample is achieved in a separation time lying in a range from 30 to 60 seconds.

After step (c) portions of the liquid components 332, 336 of the blood samples can be collected by means of a pipetting needle introduced through the pipetting openings 89 of cover 82 while the entire non-liquid components 322, 326 of the blood samples 312, 316 and portions of the liquid components 332, 336 of the blood samples 312, 316 are retained by layer 84 of the porous material.

The spatial distribution of the liquid component 33 and the non-liquid components 32 of blood sample 31 during axial centrifugation of container body 41 according to step (b) of the above described method and as schematically represented by Fig. 14 is similar to the spatial distribution shown by Fig. 5 for the where container body is a sample tube 11 with the only difference that the interior of container body 41 is subdivided in compartments 51-58, whereas the interior of sample tube 11 is not. At the end of the axial centrifugation of container body 41 according to step (b), the non-liquid components 322, 326 of the blood sample in any of compartments 51-58 occupy a segment of an outer portion 84a of layer 84 of porous material, whereas a first portion of the liquid components 332, 336 of the blood sample occupies an inner portion 84b of layer 84 of porous material and a second portion of the liquid components occupy spaces each of which lies between mesh 86 and a space occupied by air in the interior of a compartment 51-58 of container body 41.

In one embodiment of the above described method, each of the blood samples 312, 316 introduced into compartments 52, 56 of container 41 by pipetting through pipetting openings 88 of cover 82 is obtained by means of a blood collection tube that contains a vacuum, this tube contains a coagulation preventing agent, and the blood sample 312, 316 is introduced into the blood collection tube by venipuncture. In this case the liquid components 332, 336 separated by the above described method are blood plasma.

In another embodiment of the above described method, each of the blood samples 312, 316 introduced into compartments 52, 56 of container 41 by pipetting through pipetting openings 88 of cover 82 is obtained by means of a blood collection tube that contains a vacuum, this tube contains no coagulation preventing agent or contains a coagulation promoting agent, and the blood sample 312, 316 is introduced into the blood collection tube by venipuncture. In this case the liquid component 332, 336 separated by the above described method is blood serum.

After step (c) container body 41 and the separated liquid components 332, 336 contained in the compartments 52, 56 of container body 41 are usually kept at a suitable temperature until container body 41 and its contents are transferred to a clinical diagnostic analyzer apparatus for analysis of the liquid components 332, 336. For this purpose, the liquid components 332, 336 of the blood samples are aspirated from the interior of a compartment 51-58 of container body 41 by means of a pipetting needle which is introduced into the compartments of container body through the pipetting openings 89 and aspirates samples of the liquid components 332, 336, and transfers them e.g. to reaction cuvettes of the clinical diagnostic analyzer apparatus.

In a preferred embodiment, step (b) is carried out with a centrifuge which is part of a clinical diagnostic analyzer which has an automatic pipetting unit and the blood samples to be processed are introduced by the latter pipetting unit through openings 88 of cover 82 of container 41 into compartments 52, 56 of this container, and after the separation of the liquid and non-liquid components of the blood samples, the pipetting unit of the analyzer aspirates samples of the liquid components from the lower portions of compartments 52, 56 of container 41, and this samples are analyzed in the clinical diagnostic analyzer.

In a preferred embodiment, in step (a) of the above described method a blood sample is loaded into each of the compartments 51-58 of container body 41.

### FOURTH EXAMPLE OF A DEVICE ACCORDING TO THE INVENTION

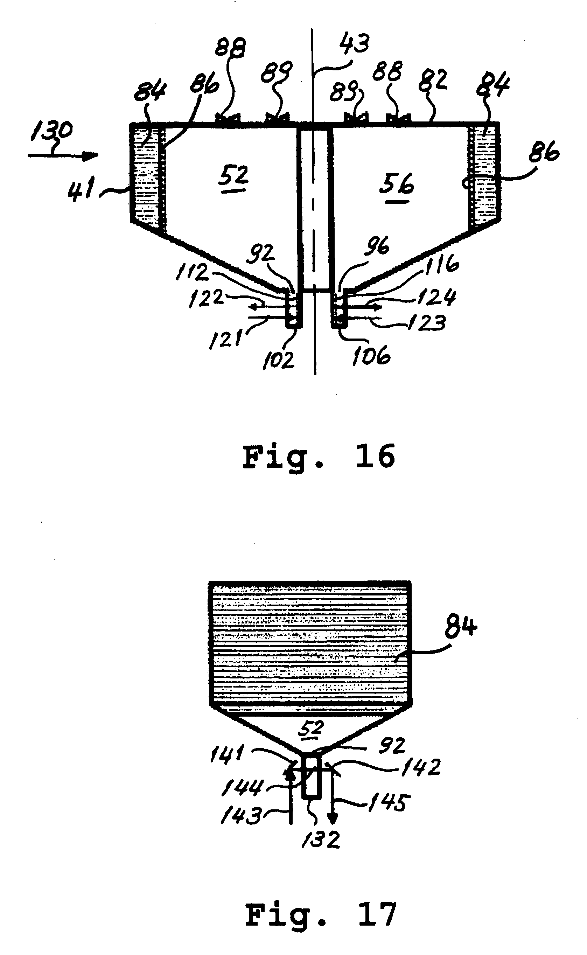

A fourth example of a device according to the invention is described hereinafter with reference to Fig. 16.

The structure of this fourth example is similar to the structure of the third example described above with reference to Figures 10 to 12, but in this fourth example each of the compartments 51-58 of container body 41 comprises in addition a cup 102, 106, which is fluidically connected with the interior of that compartment 52, 56 through an opening 92, 96 in the bottom wall 49 of the container body 41. Each of cups 102, 106 is configured and dimensioned for enabling photometric measurement of a liquid contained in that cup. As shown by Fig. 16, each of cups 102, 106 has plane parallel side walls which extend downwards from opening 92, 96 in the bottom wall of the corresponding compartment 52, 56.

In a preferred embodiment of the device shown by Fig. 16 a mirror 112, 116 is so arranged in each of the cups 102, 106 that when an incident light beam 121, 123 is transmitted towards that cup a light beam 122, 124 is reflected by mirror 112, 116. The arrangement of a mirror 112, 116 in each of cups 102, 106 enables a photometric measurement of a liquid contained in that cup 102, 106.

### FIFTH EXAMPLE OF A DEVICE ACCORDING TO THE INVENTION

A fifth example of a device according to the invention is described hereinafter with reference to Fig. 17. This fifth is a variant of the device shown in Fig. 16. Fig. 17 shows a front view seen from the direction indicated by arrow 130 of only one of the compartments of the device and shows a cup 132 which is fluidically connected with compartment 52 and which is suitable for performing photometric measurements.

As shown by Fig. 17, each of the cups 132 of the device has plane parallel side walls which extend downwards from opening 92 in the bottom wall of the corresponding compartment 52.

In a preferred embodiment of the device shown by Fig. 17 mirrors 141 and 142 are so arranged with respect to each of the cups 132 that when an incident light beam 143 is transmitted towards mirror 141, a corresponding light beam 144 reflected by mirror 141 passes through cup 132 and is reflected by mirror 142 which emits a corresponding reflected light beam 145. The arrangement of a mirrors 141, 142 associated with each one of cups 132 enables a photometric measurement of a liquid contained in that cup 132.

### FOURTH EXAMPLE OF A METHOD ACCORDING TO THE INVENTION

An fourth example of a method according to the invention for separating a liquid component from a blood sample makes use of the device described above with reference to Fig. 16 or a device described above with reference to Fig. 17 and comprises the following steps:
(a) introducing blood samples 312, 316 (as shown in Fig. 13) into respective compartments 52, 56 of the container body 41 of a device according to the above described third example of a device according to the invention,
(b) rotating the container body 41 about its symmetry axis 43 (as shown by Fig. 14) at a predetermined speed and in a sense shown by arrow 50 for separating the liquid components 332, 336 of the blood samples 312, 316 from the non-liquid components 322, 326 thereof, and
(c) stopping the rotating of the container body 41, thereby allowing the liquid components 332, 336 of the blood samples 312, 316 to flow towards the central and bottom regions 72, 76 and into cups 102, 106, 132 of the respective compartments 52, 56 of the container body 41, whereas the entire non-liquid components 322, 326 of the blood samples 312, 316 and portions of the liquid components 332, 336 of the blood samples 312, 316 are retained by layer 84 of the porous material.

Step (b) is carried out with a rotation speed adjusted to a value in a range between 500 and 10000 rpm. The time required for the separation depends from the rotation speed. A decrease of the time required for the separation is obtained by increasing the rotation speed. In the case of centrifugation of the container body 41 the following are examples of rotation speeds used and of the values of the separation time achieved:

| | | |
|---|---|---|
| Rotation speed | 500 rpm | 10000 rpm |
| Separation time | 20 minutes | 15 seconds. |

With a rotation speed of 10000 rpm, separation of platelet-free plasma of a blood sample is achieved in a separation time lying in a range from 30 to 60 seconds.

After step (c) a portion of the liquid components 332, 336 of the blood sample can be collected by pipetting through the pipetting openings 89 of cover 82 while the entire non-liquid components 322, 326 of the blood samples 312, 316 and portions of the liquid components 332, 336 of the blood samples 312, 316 are retained by layer 84 of the porous material.

In this fourth example of a method according to the invention a portion of the liquid component 332, 336 separated by the above described method is contained in cup 102, 106 or 132 and enables photometric measurements of the liquid component 332, 336 contained in this cup.

In one embodiment of the above described method, each of the blood samples 312, 316 introduced into compartments 52, 56 of container 41 by pipetting through pipetting openings 88 of cover 82 is obtained by means of a blood collection tube that contains a vacuum, this tube contains a coagulation preventing agent, and the blood sample 312, 316 is introduced into the blood collection tube by venipuncture. In this case the liquid component 332, 336 separated by the above described method is blood plasma.

In another embodiment of the above described method, each of the blood samples 312, 316 introduced into compartments 52, 56 of container 41 by pipetting through pipetting openings 88 of cover 82 is obtained by means of a blood collection tube that contains a vacuum, this tube contains no coagulation preventing agent or contains a coagulation promoting agent, and the blood sample 312, 316 is introduced into the blood collection tube by venipuncture. In this case the liquid component 332, 336 separated by the above described method is blood serum.

In a preferred embodiment, in step (a) of the above described method a blood sample is loaded into each of the compartments 51-58 of container body 41.

Sample integrity checks including measurement of serum indices can be performed by photometric measurement of the serum sample contained in cup 102, 106 or 132.

The further processing of the contents of container body 41 after step (c) is e.g. as described above in the third example of a method according to the invention.

### SECOND EXAMPLE OF AN ANALYTICAL APPARATUS ACCORDING TO THE INVENTION

A second example of an analytical apparatus according to the invention is e.g. a clinical diagnostic analyzer apparatus comprising a centrifuge to receive the device as described above with reference to Figures 10 to 12 and an arrangement for centrifugating the container body 41 of such a device about its symmetry axis 43.

In a preferred embodiment of this apparatus it comprises a device as described above with reference to Figures 16 and 17 and electro-optical means for performing photometric measurements of the contents of the cup 102, 106 or 132 of that device.

Although preferred embodiments of the invention have been described using specific terms, such description is for illustrative purposes only, and it is to be understood that changes and variations obvious to the skilled artisan are to be considered within the scope and spirit of the subject application, which is only to be limited by the claims that follow and their equivalents.

## Claims

1. A device for separating and collecting a liquid component of a blood sample, said device comprising
(a) a container body (11, 41) having a symmetry axis (13, 43) and consisting of
(a.1) an upper portion (14, 44) having a cylindrical side wall (15, 45), and an upper opening (16, 46), and
(a.2) a lower portion (17, 47) having a bottom region (18, 48) and a bottom wall (19, 49), and
(c) a layer of porous material (24, 84) arranged within said container body (11), the outer surface of said porous material (24, 84) being in contact with and covering at least partially the inner surface of said cylindrical side wall (15, 85), said porous material (24, 84) being suitable for retaining non-liquid components (32) of said blood sample (31, 312).

2. A device according to claim 1, wherein said container body is a one-piece container body.

3. A device according to claim 1, said device further comprising a mesh or stent (26, 86) which is in contact with and extends at least partially over the inner surface of said of layer of porous material (24, 84).

4. A method of separating a liquid component from a blood sample, said method comprising:
(a) introducing a blood sample (31) into container body (11, 41) of a device according to any of claims 1 to 3
(b) rotating said container body (11, 41) about its symmetry axis (13, 43) at a predetermined speed for separating the liquid component (33) of the blood sample (31) from the non-liquid components (32) thereof, and
(c) stopping said rotating of said container body (11, 41), thereby allowing the liquid component of the blood sample to flow towards the central and lower part of the container body (11, 41), whereas the entire non-liquid components (32) of the blood sample (31) and a portion of the liquid component (33) of the blood sample (31) are retained by the layer (24) of porous material.

5. A clinical diagnostic analyzer apparatus comprising
(a) a device according to any of claims 1 to 3, and
(b) means for centrifugating said container body (11, 41) of said device about its symmetry axis (13, 43).

6. A device according to any of claims 1 to 3, wherein said container body is a sample tube (11), said sample tube (11) having a symmetry axis (13), a cylindrical side wall (15), an upper opening (16), a bottom region (18), and a bottom wall (19),
said device further comprising
(a) a cap (21) which closes the upper opening (16) of said sample tube (11), said cap being piercable by a pipetting needle, and
(b) a layer (24) of a porous material arranged within said sample tube (11), the outer surface of said layer (24) being in contact with and covering at least partially the inner surface (25) of said cylindrical side wall (15), said layer (24) of porous material being suitable for retaining non-liquid components (32) of said blood sample.

7. A device according to claim 6, wherein said layer (24) of porous material is made of an open cell foam, a foamed rubber, a fleece, a mat, a honeycomb-like material or the like.

8. A device according to any of claims 6 or 7, said device further comprising a tubular mesh or stent (26) which is in contact with and extends at least partially over the inner surface of said layer (24) of porous material.

9. A device according to claim 8, wherein said tubular mesh or stent (26) is made of a plastic material.

10. A device according to any of claims 6 to 9, wherein said sample tube (11) contains a coagulation preventing agent.

11. A device according to any of claims 6 to 9, wherein said sample tube (11) contains a coagulation promoting agent.

12. A device according to any of claims 6 to 11, wherein said device further comprises a cup (22), which is fluidically connected with the interior of sample tube (11) through an opening (23) in the bottom wall (19) of the sample tube (11), said cup (22) being configured and dimensioned for enabling photometric measurement of a liquid contained in said cup (22).

13. A method of separating a liquid component from a blood sample, said method comprising:
(a) introducing a blood sample (31) into the sample tube (11) of a device according to any of claims 6 to 11
(b) rotating said sample tube (11) about its symmetry axis (13) at a predetermined speed for separating the liquid component (33) of the blood sample (31) from the non-liquid components (32) thereof, and
(c) stopping said rotating of said sample tube (11), thereby allowing the liquid component of the blood sample to flow towards the central and lower part of the sample tube (11), whereas the entire non-liquid components (32) of the blood sample (31) and a portion of the liquid component (33) of the blood sample (31) are retained by the layer (24) of porous material.

14. A method for separating a liquid component from a blood sample, said method comprising:
(a) introducing a blood sample (31) into a sample tube (11) of a device according to claim 12,
(b) rotating said sample tube (11) about its symmetry axis (13) at a predetermined speed for separating the liquid component (33) of the blood sample (31) from the non-liquid components (32) thereof, and
(c) stopping said rotating of said sample tube (11), thereby allowing the liquid component of the blood sample to flow towards the central and lower part of the sample tube (11) and into said cup (22), whereas the entire non-liquid components (32) of the blood sample (31) and a portion of the liquid component (33) of the blood sample (31) are retained by the layer (24) of porous material.

15. A method for carrying out sample integrity checks including measurement of serum indices of a blood sample, said method comprising
(a) separating the serum component of said blood sample (31) by a method according to any of claims 13 or 14, and
(b) effecting a photometric measurement of the serum sample contained in a cup (22) of a device according to claim 12.

16. A clinical diagnostic analyzer apparatus comprising
(a) a device according to any of claims 6 to 12, and
(b) means for centrifugating the sample tube (11) of said device about its symmetry axis (13).

17. A clinical diagnostic analyzer apparatus comprising
(a) a device according to claim 12,
(b) a centrifuge for centrifugating the sample tube (11) of said device about its symmetry axis (13), and
(c) electro-optical means for performing photometric measurements of the contents of the cup (22) of said device.

18. A device according to any of claims 1 to 3, said device comprising
(a) a container body (41) having a symmetry axis (43) and consisting of
(a.1) an upper portion (44), said upper portion having a cylindrical side wall (45) and an upper opening (46), and
(a.2) a lower portion (47) having the shape of a truncated cone, said lower portion having and a conical side wall (48) and a bottom wall (49),
the interior of said container body (41) being divided into a plurality of compartments (51-58) by radially oriented partition walls (91-98), each of said compartments (52, 56) having a bottom region (72, 76),
(b) a layer (84) of a porous material arranged within each of said compartments (51-58), the outer surface of said layer (84) being in contact with and covering at least partially the inner surface (85) of the portion of said cylindrical side wall (45) which belongs to said compartment, said layer (84) of porous material being suitable for retaining non-liquid components (32) of said blood sample, and
(c) a cover (82) of the said upper opening (46) of said container body (41), said cover (82) being permanently connected to said container body (41), and said cover (82) having for each of said compartments (51-58) at least one pipetting opening (88, 89) which allows passage of a pipetting needle therethrough for pipetting a blood sample into said compartment (51-58) and for pipetting out of said compartment (51-58) said liquid component of that blood sample (332-338).

19. A device according to claim 18, wherein said container body is a one-piece container body.

20. A device according to claim any of claims 18 or 19, wherein said layer (84) of porous material is made of an open cell foam, a foamed rubber, a fleece, a mat, a honeycomb-like material or the like.

21. A device according to any of claims 18 to 20, said device further comprising a mesh or stent (86) which is in contact with and extends at least partially over the inner surface of said layer (84) of porous material.

22. A device according to claim 21, wherein said mesh or stent (86) is made of a plastic material.

23. A device according to any of claims 18 to 22, wherein each of said compartments (52, 56) of said container body (41) contains a coagulation preventing agent.

24. A device according to any of claims 18 to 22, wherein each of said compartments (52, 56) of said container body (41) contains a coagulation promoting agent.

25. A device according to any of claims 18 to 24, wherein said device further comprises a plurality of cups (102, 106), each of which is located below one of the compartments (52, 56) of said container body (41) and is fluidically connected with the interior of said compartment (52, 56)through an opening (91-98) in the bottom wall (49) of the lower portion (45) of said container body (41), said cups (102, 106) being configured and dimensioned for enabling a photometric measurement of a liquid contained therein.

26. A method for separating a liquid component from a blood sample, said method comprising:
(a) introducing blood samples (312, 316) into respective compartments (52, 56)of the container body (41) of a device according to any of claims 18 to 24,
(b) rotating the container body (41) about its symmetry axis (43) at a predetermined speed for separating the liquid components (332, 336) of the blood samples (312, 316) from the non-liquid components (322, 326) thereof, and
(c) stopping the rotating of the container body (41), thereby allowing the liquid components (332, 336) of the blood samples (312, 316) to flow towards the central and bottom regions (72, 76) of the respective compartments (52, 56) of the container body (41), whereas the entire non-liquid components (322, 326) of the blood samples (312, 316) and a portion of the liquid components (332, 336) of the blood samples (312, 316) are retained by layer (84) of the porous material.

27. A method for separating a liquid component from a blood samples, said method comprising:
(a) introducing blood samples (312, 316) into respective compartments (52, 56) of the container body (41) of the device of claim 25,
(b) rotating the container body (41) about its symmetry axis (43) at a predetermined speed for separating the liquid components (332, 336) of the blood samples (312, 316) from the non-liquid components (322, 326) thereof, and
(c) stopping the rotating of the container body (41), thereby allowing the liquid components (332, 336) of the blood samples (312, 316) to flow towards the central and bottom regions (72, 76) and into said cups (102, 106, 132) of the respective compartments (52, 56) of the container body (41), whereas the entire non-liquid components (322, 326) of the blood samples (312, 316) and portions of the liquid components (332, 336) of the blood samples (312, 316) are retained by layer (84) of the porous material.

28. A method for carrying out sample integrity checks including measurement of serum indices of a blood sample, said method comprising
(a) separating the serum component of said blood sample (312, 316) by a method according to claim 27, and
(b) effecting a photometric measurement of the serum sample contained in a cup (102, 106, 132) of a device according to claim 23.

29. A clinical diagnostic analyzer apparatus comprising
(a) a device according to any of claims 18 to 24, and
(b) means for centrifugating the container body (41) of said device about the symmetry axis (43) of said container body (41).

30. A clinical diagnostic analyzer apparatus comprising
(a) a device according to claim 25,
(b) means for centrifugating the container body (41) of said device about the symmetry axis (43) of said container body (41), and
(c) electro-optical means for performing photometric measurements of the contents of the cups (102, 106, 132) of said device.
